# EUROPEAN PATENT APPLICATION

(11) **EP 3 103 863 A1**
(43) Date of publication of application: **14.12.2016**
(21) Application number: 16173254.0
(22) Date of filing: 07.06.2016
(51) Int. Cl.: C12M 1/34, C12M 1/36, C12M 1/33, C12M 1/00

(54) **CONTROLLING DRY MATTER CONTENT OF A PROCESS STREAM COMPRISING A BIOLOGICAL SUBSTRATE**

(30) Priority: 09.06.2015 SE 1550765
(71) Applicant: Cellwood Machinery AB, 571 32 Nässjö (SE)
(72) Inventor: EK, Peter, 571 40 Nässjö (SE)
(74) Representative: Kransell & Wennborg KB

(57) **Abstract**

The present disclosure relates to a process arrangement for controlling dry matter content of a process stream comprising a biological substrate. The arrangement comprises a mixing unit 1 configured for mixing dry and liquid matter of the process stream. The arrangement also comprises a separating unit 2, configured for being downstream of the mixing unit and for separating heavy phase particles from the process stream. The arrangement also comprises a concentrating unit 3, configured for being downstream of the separating unit and for removing process water from the process stream, thereby concentrating the dry matter of the biological substrate in said process stream. The arrangement also comprises a first recirculation loop 13a arranged for recirculating at least a part of the process water from the concentrating unit to the process stream upstream of the separating unit. The arrangement also comprises a second recirculation loop 14a arranged for recirculating at least a part of the process stream with concentrated dry matter from downstream of the concentrating unit to the process stream upstream of the separating unit. The arrangement also comprises a flow detector 41 for measuring in-line the dry matter content of the process stream between the mixing unit and the separating unit. The arrangement also comprises a controller 33 for controlling the recirculation in the first recirculation loop based on the measured in-line the dry matter content, and for controlling the recirculation in the second recirculation loop based on the measured in-line the dry matter content.

## Description

### TECHNICAL FIELD

The present disclosure relates to a process and an arrangement for processing a stream of a biological substrate such as food waste or manure comprising removal of heavy phase particles such as stones, sand or glass.

### BACKGROUND

Biological waste may be used for e.g. producing biogas in which case the waste is fed to an anaerobic digester for formation of methane gas, or for another process using a biosubstrate. Wear and tear of equipment as well as sedimentation in buffer tanks is a concern in most pretreatment plants causing major disruptions and high costs to recover. In order to improve the efficiency of the plant, heavy phase contaminants such as sand and glass (e.g. contaminating food waste from households or supermarkets and which tend to accumulate in a plant over time), as well as excess water, is removed from the biological waste before entering the digester. The excess water may be removed by pressing it from the waste, and the contaminants may be removed by a separator. Lately, the amount of sand in manure has increased due to sand increasingly being used as bedding for cows.

### SUMMARY

A problem with the separation of heavy phase particles by means of a separator has been observed in that the richness, i.e. the dry matter (DM) content, of the biological waste substrate may vary over time in a process stream, depending on type of substrate and which batch it originates from. If the biological substrate e.g. is liquid manure, the dry matter content may vary depending on whether the manure is from cow or pig, or from which farm or day it originates from. The separating unit may only efficiently separate the heavy phase particles from a process stream when the dry matter (also called solid matter) content of the stream is within a certain range, or the separator may have to be recalibrated when the dry matter content of the stream changes.

Thus, it is an objective of the present invention to provide an improved process for controlling the dry matter content of the process stream comprising a biological substrate to control fluidity and improve the efficiency of the separating unit.

According to an aspect of the present invention, there is provided a process arrangement for controlling dry matter content of a process stream comprising a biological substrate. The arrangement comprises a mixing unit configured for mixing dry and liquid matter of the process stream. The arrangement also comprises a separating unit, configured for being downstream of the mixing unit and for separating heavy phase particles from the process stream. The arrangement also comprises a concentrating unit, configured for being downstream of the separating unit and for removing process water from the process stream, thereby concentrating the dry matter of the biological substrate in said process stream. The arrangement also comprises a first recirculation loop arranged for recirculating at least a part of the process water from the concentrating unit to the process stream upstream of the separating unit. The arrangement also comprises a second recirculation loop arranged for recirculating at least a part of the process stream with concentrated dry matter from downstream of the concentrating unit to the process stream upstream of the separating unit. The arrangement also comprises a flow detector for measuring in-line the dry matter content of the process stream between the mixing unit and the separating unit. The arrangement also comprises a controller for controlling the recirculation in the first recirculation loop based on the measured in-line the dry matter content, and for controlling the recirculation in the second recirculation loop based on the measured in-line the dry matter content.

According to another aspect of the present invention, there is provided a process for controlling dry matter content of a process stream comprising a biological substrate. The process comprises measuring in-line, by means of a flow detector, the dry matter content of the process stream downstream of a mixing unit and upstream of a separating unit. The process also comprises controlling, based on the measured dry matter content, a first recirculation flow in a first recirculation loop recirculating at least a part of process water removed from the process stream in a concentrating unit to the process stream upstream of the separating unit. The process also comprises controlling, based on the measured dry matter content, a second recirculation flow in a second recirculation loop recirculating at least a part of the process stream with concentrated dry matter from downstream of the concentrating unit to the process stream upstream of the separating unit.

By means of the first and second recirculation loops, and the control of the recirculation flows therein, the dry matter content of the process stream being fed to the separating unit may be automatically controlled within a predetermined desired range, optimising the efficiency of the separating unit.

The mixing unit may be any type of mixing unit e.g. a tank stirred by means of a paddle, propeller or the like, or a section of piping conducting the process stream in which the stream is mixed by means of turbulent flow. Either of the first and second recirculation loops may recirculate its flow to the mixing unit (e.g. tank) or to piping for the process stream between the mixing unit and the separating unit.

The separating unit may be any type of separating unit suitable for separating the heavy phase particles from the process stream, e.g. a cyclone such as a high-density cleaner or hydrocyclone.

The concentrating unit may be any type of concentrating unit suitable for removing process water from the process stream, e.g. a press such as a screw press, a decanter, a belt thickener or an evaporator/distiller.

The controller may comprise one or several control units for controlling the respective flows in the first and second, and the optional third, recirculation loops.

The biological substrate may comprise biological waste such as food waste and/or (animal) manure e.g. cow, pig or avian/poultry manure.

The heavy phase particles may comprise stones, sand and/or glass.

In some embodiments, the process arrangement also comprises a chopping unit for chopping up and homogenising the biological substrate upstream of the mixing unit, making the biological substrate more easily mixable and suitable for forming the process stream.

In some embodiments, the process arrangement also comprises a third recirculation loop arranged for recirculating at least a part of the process stream output from the separating unit to the process stream upstream of the separating unit. Thus, a part of the stream downstream of the separating unit may be recirculated and pass again through the separating unit in order to improve the separation, i.e. to remove a larger percentage of the heavy phase particles in the process stream. For instance, half or 2/3 of the output stream may be recirculated.

In some embodiments, the controller is configured for controlling the recirculation in the first recirculation loop by acting on a first recirculation valve controlling the flow through the first recirculation loop. The first valve may e.g. be closed if the process stream according to the flow detector is too diluted (i.e. contains too much water per volume already), but be open (fully or to varying degrees) if the process stream according to the flow detector is too rich (i.e. contains too much dry matter and too little water per volume). Additionally or alternatively, the controller is configured for controlling the recirculation in the second recirculation loop by acting on a second recirculation valve controlling the flow through the second recirculation loop. The second valve may e.g. be closed if the process stream according to the flow detector is too rich (i.e. contains too much dry matter and too little water per volume), but be open (fully or to varying degrees) if the process stream according to the flow detector is too diluted (i.e. contains too much water per volume).

It is to be noted that any feature of any of the aspects may be applied to any other aspect, wherever appropriate. Likewise, any advantage of any of the aspects may apply to any of the other aspects. Other objectives, features and advantages of the enclosed embodiments will be apparent from the following detailed disclosure, from the attached dependent claims as well as from the drawings.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated. The use of "first", "second" etc. for different features/components of the present disclosure are only intended to distinguish the features/components from other similar features/components and not to impart any order or hierarchy to the features/components.

### BRIEF DESCRIPTION OF THE DRAWINGS

Embodiments will be described, by way of example, with reference to the accompanying drawings, in which:

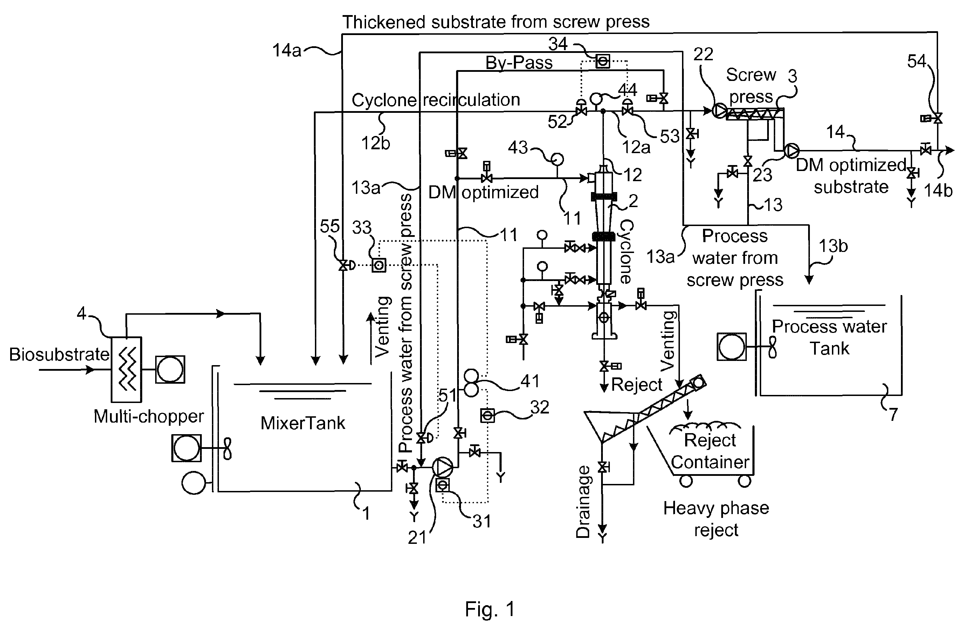

Fig 1 is a schematic process chart illustrating embodiments of the process arrangement of the present invention.

### DETAILED DESCRIPTION

Embodiments will now be described more fully hereinafter with reference to the accompanying drawing, in which certain embodiments are shown. However, other embodiments in many different forms are possible within the scope of the present disclosure. Rather, the following embodiments are provided by way of example so that this disclosure will be thorough and complete, and will fully convey the scope of the disclosure to those skilled in the art. Like numbers refer to like elements throughout the description. Figure 1 schematically illustrates an example of a process arrangement in accordance with the present invention.

The process stream (could also be called flow) runs from the mixing unit 1 via the separating unit 2 to and through the concentrating unit 3. Counter stream flows are called recirculation flows.

Figure 1 is a schematic process chart illustrating embodiments of the process arrangement of the present invention.

A biosubstrate, e.g. manure or food waste, having a dry matter content of e.g. 3-12% by weight, may be homogenized in a multi-chopper 4 or other homogenizing apparatus. The homogenized biosubstrate may be transferred to a mixing unit 1, here in the form of a mixer tank. The mixer tank 1 is connected via piping 11 and a pump 21 (controlled by a control unit 31, and possibly a control unit 32 based on a flow rate measured by the detector 41) to a separating unit 2, here in the form of a cyclone.

The cyclone is configured for separating heavy-phase particles from the process stream from the mixer tank via the piping 11. Bypass piping may also be provided for allowing a part or all of the process stream of piping 11 to bypass the separating unit 2, e.g. based on the flow rate in the piping 11 at the input to the separating unit 2 as measured by the detector 43. The output from the separating unit 2 is via piping 12 and 12a transferred to the concentrating unit 3, here in the form of a screw press. The heavy-phase particles separated from the process stream in the separating unit 2 leaves the separating unit as reject to a reject container. A part or all of the separating unit output may be recirculated to the mixing unit 1 via the piping 12b. Valves 52 and 53 are controlled by the control unit 34 for regulating the flow in pipes 12a and 12b, respectively, e.g. based on measurements of the flow detector 44.

The process stream may be fed into the concentrating unit 3, e.g. by means of a pump 22. The concentrating unit removes some of the process water from the process stream, to produce a process stream of thickened substrate (with a higher DM content) and a process water flow. All or a part of the process water may be transferred to a process water tank 7, e.g. for further use in other processes, via the piping 13 and 13b. However, at least a part of the process water flow may be recirculated to the process flow, in the mixing unit 1 or between the mixing unit and the separating unit 2, via the piping 13a (forming part of the first recirculation loop as discussed herein). A flow detector 41 measures the DM content, and possibly also the flow rate (volume per time), in the piping 11 for the process stream from the mixing unit 1 to the separating unit 2. A control unit 33 controls a valve 51 of the first recirculation loop 13a, based on the readings from the flow detector 41, to control the amount of recirculated process water in view of the DM content in the process stream in the piping 11.

The thickened biosubstrate of the process stream outputted from the concentrating unit 3 flows, e.g. by means of the pump 23, in the piping 14 and 14b to e.g. an anaerobic digestion chamber or another process using biosubstrate. By means of the concentrating unit 3, the excess water has been removed from the biosubstrate in the process stream, and by means of the separating unit 2, the heavy-phase particles have also been removed, providing a biosubstrate which is more suitable for a process using biosusbtrate as starting material. However, all or a part of the thickened substrate may be recirculated to the process stream, in the mixing unit 1 or between the mixing unit and the separating unit 2, via the piping 14a (forming part of the second recirculation loop as discussed herein). A control unit 33, possibly the same control unit controlling the recirculation of the process water, controls a valve 55 of the second recirculation loop 14a, based on the readings from the flow detector 41, to control the amount of recirculated thickened substrate in view of the DM content in the process stream in the piping 11.

By means of the first and second recirculation loops 13a and 14a, the DM content of the input feed to the separating unit 2 may be optimized for improved operation of the separating unit and thus improved separation of heavy-phase particles.

The present disclosure has mainly been described above with reference to a few embodiments. However, as is readily appreciated by a person skilled in the art, other embodiments than the ones disclosed above are equally possible within the scope of the present disclosure, as defined by the appended claims.

## Claims

1. A process arrangement for controlling dry matter content of a process stream comprising a biological substrate, the arrangement comprising:
a mixing unit (1) configured for mixing dry and liquid matter of the process stream;
a separating unit (2), configured for being downstream of the mixing unit and for separating heavy phase particles from the process stream;
a concentrating unit (3), configured for being downstream of the separating unit and for removing process water from the process stream, thereby concentrating the dry matter of the biological substrate in said process stream;
a first recirculation loop (13a) arranged for recirculating at least a part of the process water from the concentrating unit to the process stream upstream of the separating unit;
a second recirculation loop (14a) arranged for recirculating at least a part of the process stream with concentrated dry matter from downstream of the concentrating unit to the process stream upstream of the separating unit;
a flow detector (41) for measuring in-line the dry matter content of the process stream between the mixing unit and the separating unit; and
a controller (33) for automatically controlling the recirculation in the first recirculation loop based on the measured in-line the dry matter content, and for controlling the recirculation in the second recirculation loop based on the measured in-line the dry matter content.

2. The process arrangement of claim 1, further comprising:
a chopping unit (4) for chopping up and homogenising the biological substrate upstream of the mixing unit.

3. The process arrangement of claim 1 or 2, further comprising:
a third recirculation loop (12b) arranged for recirculating at least a part of the process stream output from the separating unit (2) to the process stream upstream of the separating unit.

4. The process arrangement of any preceding claim, wherein the controller (33) is configured for controlling the recirculation in the first recirculation loop (13a) by acting on a first recirculation valve (51) controlling the flow through the first recirculation loop and/or for controlling the recirculation in the second recirculation loop (14a) by acting on a second recirculation valve (55) controlling the flow through the second recirculation loop.

5. The process arrangement of any preceding claim, wherein the separating unit (2) is a cyclone.

6. The process arrangement of any preceding claim, wherein the concentrating unit (3) is a press e.g. a screw press.

7. The process arrangement of any preceding claim, wherein the biological substrate is any of food waste and manure.

8. The process arrangement of any preceding claim, wherein the heavy phase particles comprise stones, sand and/or glass.

9. A process for controlling dry matter content of a process stream comprising a biological substrate, the process comprising:
measuring in-line, by means of a flow detector (41), the dry matter content of the process stream downstream of a mixing unit (1) and upstream of a separating unit (2);
automatically controlling, based on the measured dry matter content, a first recirculation flow in a first recirculation loop (13a) recirculating at least a part of process water removed from the process stream in a concentrating unit (3) to the process stream upstream of the separating unit (2); and automatically controlling, based on the measured dry matter content, a second recirculation flow in a second recirculation loop (14a) recirculating at least a part of the process stream with concentrated dry matter from downstream of the concentrating unit (3) to the process stream upstream of the separating unit.
